# EUROPEAN PATENT SPECIFICATION

(11) **EP 0 534 567 B1**
(45) Date of publication and mention of the grant of the patent: **27.10.1999**
(21) Application number: 92202942.6
(22) Date of filing: 25.09.1992
(51) Int. Cl.: C07D 211/46, C07D 211/94, C08K 5/3435, C07C 69/732

(54) **Stabilizers for organic materials**
Stabilisatoren für organische Materialien
Stabilisants pour matériaux organiques

(30) Priority: 25.09.1991 NL 9101619
(43) Date of publication of application: 31.03.1993
(60) Divisional application: 98202889.6
(73) Proprietor: AKCROS CHEMICALS V.O.F., NL-6041 KV Roermond (NL)
(72) Inventor: Schmets, Gerard Hubert Frans, NL-6085 AV Horn (NL); Peters, Frans Jeannette Maria, NL-6061 EL Posterholt (NL); Kroon, Erica Gertruda Arnolda, NL-6071 XM Swalmen (NL); Overveen, Jan Cornelis, NL-3925 KN Scherpenzeel (NL)
(74) Representative: De Hoop, Eric

(56) References cited:
- EP-A- 0 102 193
- EP-A- 0 389 424
- EP-A- 0 421 928
- DE-A- 2 258 752
- DE-A- 2 717 087
- US-A- 4 102 858
- US-A- 4 633 008
- CHEMICAL ABSTRACTS, vol. 73, 1970, Columbus, Ohio, US; abstract no. 14635,
- CHEMICAL ABSTRACTS, vol. 86, 1977, Columbus, Ohio, US; abstract no. 90995, MINAGAWA ET. AL. 'Light stabilizers for polymers'

## Description

The invention relates to novel stabilizers for organic materials which are liable to oxidative and/or thermal decomposition and/or decomposition under influence of actinic irradiation, such as petroleum products, e.g lubricating oils and lubricating greases, and plastics, such as polyvinylchloride, polyalkylenes and polystyrene.

In order to prevent oxidative and/or thermal decomposition (degradation) of petroleum products and plastics, such as polyvinylchloride, polyalkylenes and polystyrene, on large scale antioxidants and antiozonants are used, which depending on their chemical structure, inhibit the initiation or propagation of oxidation reactions. Especially as antioxidants that inhibit the propagation of oxidation reactions, secondary alkylaryl- and diarylamines and, in particular, hindered phenols are applied (vide Kirk-Othmer, Encyclopedia of Chemical Technology, 3rd edition part 3, page 128-148 and in particular pages 130-133).

In order to prevent decomposition under influence of actinic irradiation, in particular decomposition under influence of UV-irradiation, often so-called HALS (hindered amine light stabilizers) are applied, the most important ones of which are 2,2,6,6-tetramethyl-4-piperidinol and carboxylic acids, such as bis-(2,2,6,6-tetramethyl-4-piperidinyl)sebacate and - azelate, which are especially active in polypropylene, polyethylene, styrene polymers, polyurethanes and coatings containing acrylic polymer (vide Kirk- Othmer, Encyclopedia of Chemical Technology, 3rd edition, volume 23, page 615-627 and in particular pages 620-621 and page 624).

Although said compounds generally have good properties, continuously novel compounds of these types are developed, in order to meet the higher and higher requirements with respect to the stability of petroleum products (e.g. lubricating oil) and of polymers.

So in EP-A-89119641.2 (publication no. 366 040) hindered phenol antioxidants of formula 1 are described, wherein R is a methyl group, R₁ is an alkyl, cycloalkyl, phenyl, or aralkyl group, R₂ is hydrogen or an alkyl, cycloalkyl, phenyl or aralkyl group, R₃ is hydrogen or a methyl group, n is an integer of 1-4 or 6, wherein, if n = 2, A is preferably an alkylene dioxy group having 2-8 carbon atoms or an alkylene dioxy group interrupted by ether oxygen or a thio group.

In EP-A-88108282.0 (publ. no. 300160) the ester of 2,2,6,6-tetramethyl-4-piperidinol and behenic acid is described, which serves as stabilizer against the decomposition of polymers by actinic irradiation.

Now the object of the invention is to provide novel stabilizers with particularly favourable, improved properties.

According to a first aspect of the invention, novel compounds of formula 1 are provided, wherein R is hydrogen or a methyl group, R₁ and R₂, which may be the same or different, represent a tertiary alkyl group having 4-8 carbon atoms, R₃ is hydrogen or a methyl group, n = 2 and A is a polyol having 3-6 C-atoms, or a dipolyol having 6-12 C-atoms.

Compared to the known stabilizers of this type, these novel compounds have improved properties with respect to the stabilization against thermal and oxidative degradation of both petroleum products and plastics.

In the novel compounds of formula 1, R₁ and R₂ preferably both are a tertiary butyl group. The tertiary butyl group provides an excellent sterical hindering of the hydroxyl group of the phenol and is extremely stable itself, so that the compounds have an optimum stabilizing effect.

The polyol or di-polyol group (A) has at least one free hydroxyl group, which also contributes to the stabilizing effect of the compounds.

Preferably A is a glycerol or di-glycerol moiety. The stabilizing effect of the compound is then excellent, while the compounds themselves are stable.

According as the number of free hydroxyl groups of the polyol or dipolyol moiety is higher, the long term thermal stability improves, but this is at the expense of the colour stability of the compounds.

The novel stabilizers are included in usual amounts in the petroleum products or plastics to be stabilized that is to say amounts of 0.1-10 and preferably 0.05-5% by weight. Optimal results are generally obtained with 0.1-3 and in particular with 1-3 % by weight.

The invention is further elucidated by the following examples.

### Example I

### Preparation of diglyceryl-bis[β-(3,5-di-tertiary butyl-4-hydroxyphenyl)propionic ester] (reaction scheme A, formula 5).

A three-neck flask with stirrer, thermometer and cooler is filled with :

| | |
|---|---|
| 40 g (0.137 moles) | β-(3,5-di-tertiary butyl-4-hydroxyphenyl)-propionic acid methyl ester (I) |
| 12.95 g (0.078 mol) | diglycerine (II) |
| 2 g | titanium butoxide |

The reaction mixture was heated at 160°C for 5 hours, while the methanol formed with the reaction was continuously removed by means of distillation. Subsequently the reaction mixture was cooled down. The brown-coloured residue was incorporated into the ether. The ether layer was washed with water and dried whereafter the ether was destilled. The residue was dissolved in ethanol and purified over a column of silica gel. After distilling the ethanol a yellow-coloured viscous liquid was obtained.
Yield: 39.6 g = 85%
The NMR spectrum is in accordance with formula 5.

### Example II

The compound according to example I was applied as stabilizer in a softener-free, calcium-zinc-stabilized PVC composition

| | |
|---|---|
| S-PVC (K=68-70) | 100 parts by weight |
| Paraloid KM-334 (Impact-strenght additive | 5-8 parts by weight |
| Paraloid K-120N (Flow improver) | 0.5-1.5 parts by weight |
| Hydrocarb 95T (chalk) | 3-10 parts by weight |
| Titanium dioxide (Kronos-2220) | 3-10 parts by weight |
| Ca/Zn stabilizer | 3.5-4.5 parts by weight |
| Stabilizer (oxyd/therm., UV) | 1-3 parts by weight |

Test plates of this composition which contain the stabilizer according to example I were tested on their colour stability, and compared with test plates with an analogous composition wherein only β-(3,5-di-t-butyl-4-hydroxyphenyl)propionic-n-octadecyl ester were applied.

The colour stability with the stabilizer of Example I was, when applied in amounts between 1 and 3 parts by weight (on 100 parts by weight of PVC), in any case better than with the comparative stabilizer.

### Example III

The compound according to example I was applied as stabiliser in a softener containing film composition stabilized with Ba/Zn:

| | |
|---|---|
| S-PVC (K=65) | 100 parts by weight |
| Softener (DOP) | 20-25 parts by weight |
| Epoxized soy bean oil | 2-3 parts by weight |
| Titanium dioxide | 5-10 parts by weight |
| Stabilizer | 1.5-3 parts by weight |

Test films of this composition which contain the stabilizer of example I and films which contain a usual stabilizer pentaerythritol-tetra [β-3-(3,5-di-t-butyl-4-hydroxyphenyl)-proprionate] were tested on their resistance against thermal and oxidative degradation and the yellow coloration after the test was measured with a CIE Lab colour measuring system, manufacturer Dr. Lange.

The yellow coloration of the films with the stabilizer of Example I was less than that of the films which contain the usual stabilizer.

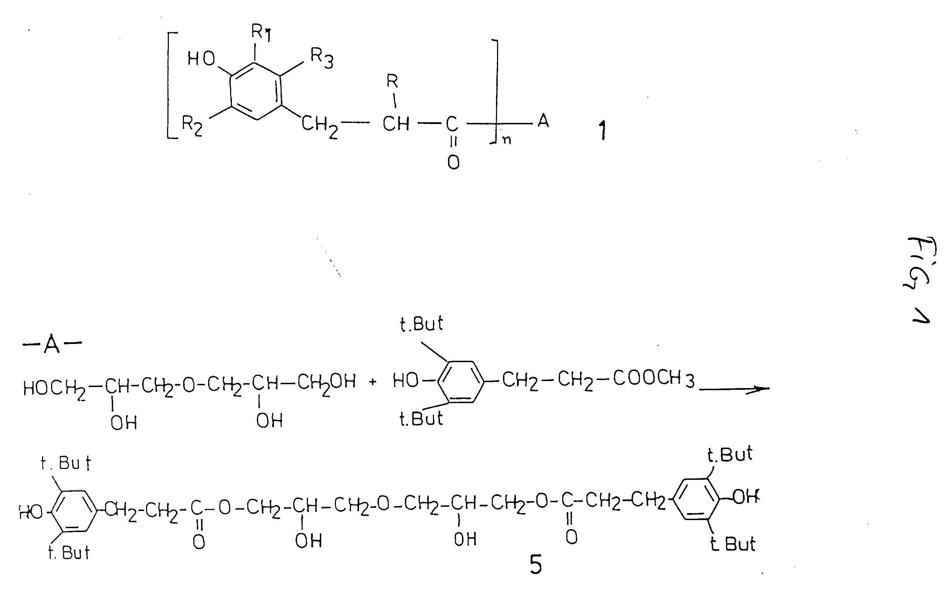

## Claims (Claims for the following Contracting State(s): AT, BE, CH, LI, DE, DK, FR, GB, GR, IE, IT, LU, MC, NL, PT, SE)

1. Stabilizer for organic materials which are liable to oxidative and/or thermal decomposition and/or decomposition under influence of actinic irradiation, of formula 1, 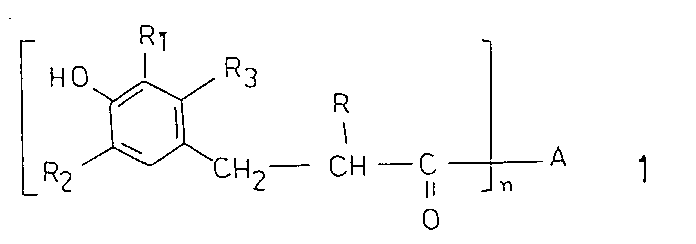 wherein
R is hydrogen or a methyl group, R₁ and R₂, which may be the same or different, represent a tertiary alkyl group having 4-8 C-atoms, R₃ is hydrogen or a methyl group, n = 2 and A is a polyol having 3-6 C-atoms or a di-polyol having 6-12 C-atoms.

2. Stabilizer according to claim 1, wherein R₁ and R₂ both are a tertiary butyl group.

3. Stabilizer according to claim 1 or 2, wherein A is a glycerol or di-glycerol moiety.

4. Stabilizer according to claim 1 or 2, wherein A is a sorbitol or di-sorbitol moiety.

5. Petroleum product or plastic material stabilized against oxidative and/or thermal decomposition and/or decomposition under influence of actinic irradiation, characterized in that it contains a compound according to any one of the preceding claims as stabilizer.

## Claims (Claims for the following Contracting State(s): ES)

1. A process for preparing a stabilizer for organic materials which are liable to oxidative and/or thermal decomposition and/or decomposition under influence of actinic irradiation, which process comprises reacting a compound of the formula 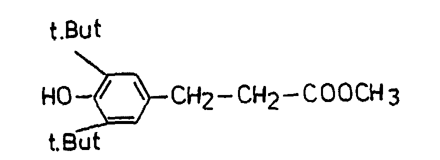 with a polyol of the formula 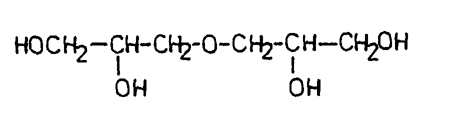 to yield a stabilizer of the formula

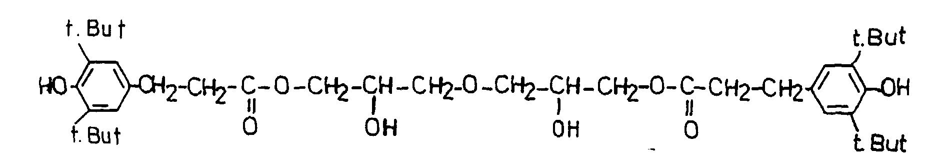

2. Petroleum product or plastic material stabilized against oxidative and/or thermal decomposition and/or decomposition under influence of actinic irradiation, characterized in that it contains as stabilizer a compound of the formula 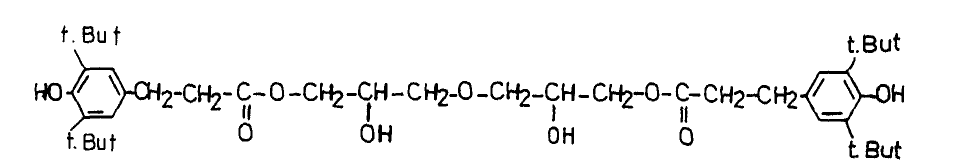 prepared by the process of claim 1.

## Patentansprüche (Patentansprüche für folgende(n) Vertragsstaat(en): AT, BE, CH, DE, DK, FR, GB, GR, IE, IT, LI, LU, MC, NL, PT, SE)

1. Stabilisator für organische Materiale, die oxidierender und/oder thermischer Zersetzung unterliegen und/oder Zersetzung unter Einfluß von aktinischer Bestrahlung, mit Formel 1, 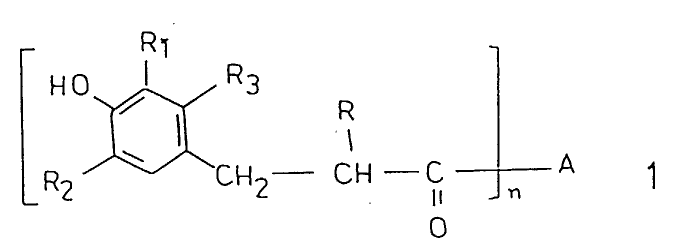 worin
R Wasserstoff oder eine Methylgruppe ist, R₁ und R₂, welche dasselbe oder verschieden sein können, eine tertiäre Alkylgruppe mit 4-8 C-Atomen darstellen, R₃ Wasserstoff oder eine Methylgruppe ist, n = 2 und A ein Polyol mit 3-6 C-Atomen oder ein Dipolyol mit 6-12 C-Atomen ist.

2. Stabilisator gemäß Anspruch 1, worin R₁ und R₂ beide eine tertiäre Butylgruppe sind.

3. Stabilisator gemäß Anspruch 1 oder 2, worin A ein Glycerol- oder Diglycerolgruppe ist.

4. Stabilisator gemäß Anspruch 1 oder 2, worin A ein Sorbitol- oder Disorbitolgruppe ist.

5. Erdölprodukt oder Kunststoffmaterial stabilisiert gegen oxidierende und/oder thermische Zersetzung und/oder Zersetzung unter Einfluß von aktinischer Bestrahlung, dadurch gekennzeichnet, daß es als Stabilisator eine Verbindung gemäß einem der vorheraehenden Ansprüche enthält.

## Patentansprüche (Patentansprüche für folgende(n) Vertragsstaat(en): ES)

1. Ein Verfahren zum Herstellen eines Stabilisators für organische Materiale, die oxidierender und/oder thermischer Zersetzung unterliegen und/oder Zersetzung unter Einfluß von aktinischer Bestrahlung, welches Verfahren eine Reaktion einer Verbindung der Formel 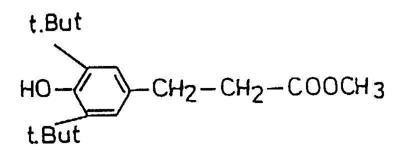 mit einem Polyol der Formel 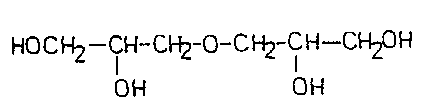 um ein Stabilisator der Formel 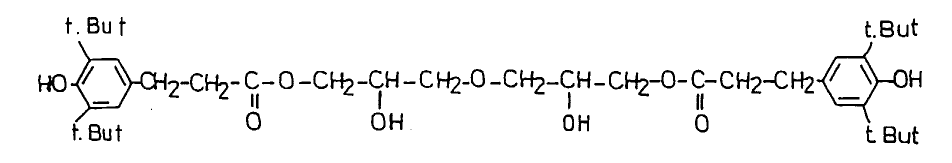 zu erhalten, umfaßt.

2. Erdölprodukt oder Kunstoffmaterial stabilisiert gegen oxidierende und/oder thermische Zersetzung und/oder Zersetzung unter Einfluß von aktinischer Bestrahlung, dadurch gekennzeichnet, daß es als Stabilisator eine Verbindung der Formel 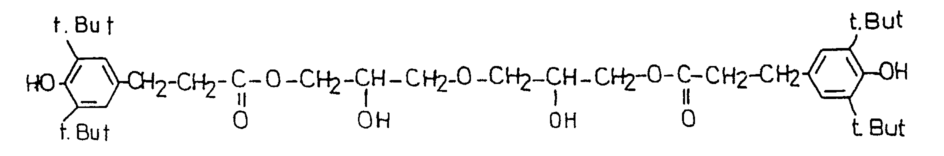 mit dem Verfahren von Anspruch 1 hergestellt, enthält.

## Revendications (Revendications pour l'(les) Etat(s) contractant(s) suivant(s): AT, BE, CH, DE, DK, FR, GB, GR, IE, IT, LI, LU, MC, NL, PT, SE)

1. Stabilisant pour des matières organiques qui sont sujettes à la décomposition oxyclative et/ou thermique et/ou à la décomposition sous l'influence d'un rayonnement actinique, de formule 1 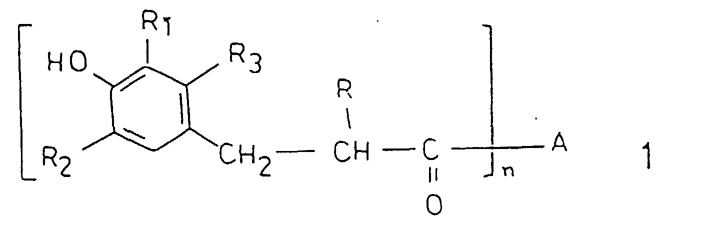 dans laquelle R est l'hydrogène ou un groupe méthyle, R₁ et R₂, qui peuvent être identiques ou différents, représentent un groupe alkyle tertiaire possédant 4-8 atomes de C, R₃ est l'hydrogène ou un groupe méthyle, n = 2 et A est un polyol possédant 3-6 atomes de C ou un dipolyol possédant 6-12 atomes de C.

2. Stabilisant selon la revendication 1, dans lequel R₁ et R₂ sont tous les deux un groupe butyle tertiaire.

3. Stabilisant selon la revendication 1 ou 2, clans lequel A est une portion de glycérol ou de diglycérol.

4. Stabilisant selon la revendication 1 ou 2, clans lequel A est une portion de sorbitol ou de disorbitol.

5. Produit pétrolier ou matière plastique stabilisé vis à vis de la décomposition oxydative et/ou thermique et/ou de la décomposition sous l'influence d'un rayonnement actinique, caractérisé en ce qu'il contient, en tant que stabilisant, un composé selon l'une quelconque des revendications précédentes.

## Revendications (Revendications pour l'(les) Etat(s) contractant(s) suivant(s): ES)

1. Un procédé de préparation d'un stabilisant pour des matières organiques qui sont sujettes à la décomposition oxyclative et/ou thermique et/ou à la décomposition sous l'influence d'un rayonnement actinique, lequel procédé consiste à faire réagir un composé de formule 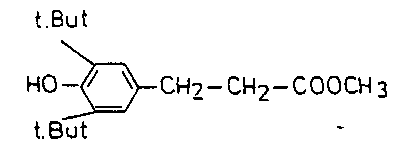 avec un polyol de formule 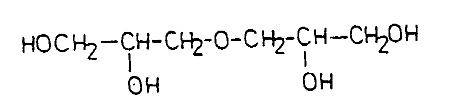 en fournissant un stabilisant de formule

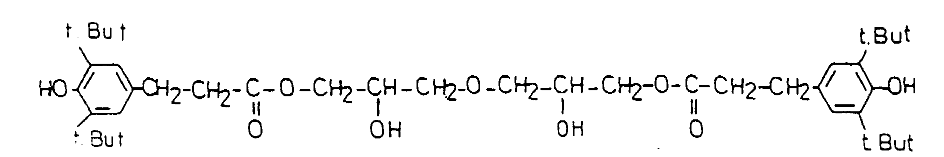

2. Produit pétrolier ou matière plastique stabilisé vis à vis de la composition oxydative et/ou thermique et/ou de la décomposition sous l'influence d' un rayonnement actinique, caractérisé en ce qu'il contient, en tant que stabilisant, un composé de formule 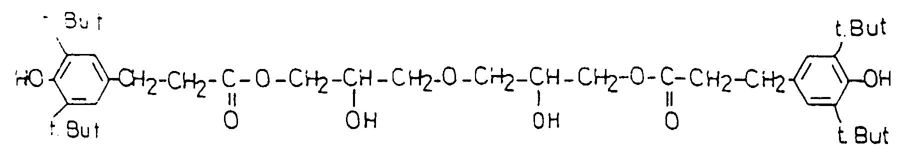 préparé par le procédé de la revendication 1.
